# EUROPEAN PATENT SPECIFICATION

(11) **EP 1 392 256 B1**
(45) Date of publication and mention of the grant of the patent: **21.04.2010**
(21) Application number: 02735338.2
(22) Date of filing: 06.05.2002
(51) Int. Cl.: A61K 9/70, A61K 31/381

(54) **IMPROVED TRANSDERMAL THERAPEUTIC SYSTEM FOR THE TREATMENT OF PARKINSON'S DISEASE**
VERBESSERTES TRANSDERMALES THERAPEUTISCHES SYSTEM FÜR DIE BEHANDLUNG DER PARKINSON-KRANKHEIT
SYSTEME THERAPEUTIQUE TRANSDERMIQUE AMELIORE POUR LE TRAITEMENT DE LA MALADIE DE PARKINSON

(30) Priority: 08.05.2001 EP 01111110
(43) Date of publication of application: 03.03.2004
(73) Proprietor: UCB Pharma GmbH, 40789 Monheim (DE); LTS LOHMANN Therapie-Systeme AG, 56626 Andernach (DE)
(72) Inventor: LAUTERBACH, Thomas, 40595 Düsseldorf (DE); SCHACHT, Dietrich, Wilhelm, 50935 Köln (DE); WOLFF, Hans-Michael, 40789 Monheim (DE); MÜLLER, Walter, 56626 Andernach (DE)
(74) Representative: HOFFMANN EITLE
(86) International application number: PCT/EP2002/004975
(87) International publication number: WO 2002/089777

(56) References cited:
- WO-A-99/49852
- DE-A- 19 940 238

## Description

### Field of the Invention

The present invention relates a Transdermal Therapeutic System (TTS) for delivering the dopamine receptor agonist rotigotine in a sufficient amount and at a sufficient rate to provide therapeutically effective treatment or alleviation of symptoms of Parkinson's disease.

### Technical Background

Parkinson's disease is believed to be primarily caused by the degeneration of dopaminergic neurons in the substantia nigra. This, in effect, results in loss of tonic dopamine secretion and dopamine-related modulation of neuronal activity in the caudate nucleus, and thus in a deficiency of dopamine in certain brain regions. The resulting imbalance of the neurotransmitters acetylcholine and dopamine eventually results in disease-related symptoms. Although usually regarded as a motor system disorder, Parkinson's Disease is now considered to be a more complex disorder that involves both motor and nonmotor systems. This debilitating disease is characterized by major clinical features including tremor, bradykinesia, rigidity, dyskinesia, gait disturbances, and speech disorders. In some patients, dementia may accompany these symptoms. Involvement of the autonomic nerve system may produce orthostatic hypotension, paroxysmal flushing, problems with thermal regulation, constipation, and loss of bladder and sphincter control. Psychological disorders such as loss of motivation and depression may also accompany Parkinson's Disease.

Parkinson's Disease is primarily a disease of middle age and beyond, and it affects both men and women equally. The highest rate of occurrence of Parkinson's Disease is in the age group over 70 years old, where Parkinson's Disease exists in 1.5 to 2.5% of that population. The mean age at onset is between 58 and 62 years of age, and most patients develop Parkinson's Disease between the ages of 50 and 79. There are approximately 800,000 people in the United States alone with Parkinson's Disease.

Early motor deficits of Parkinson's Disease can be traced to incipient degeneration of nigral dopamine-releasing cells. This neuronal degeneration produces a defect in the dopaminergic pathway that connects the substantia nigra to the striatum. As the disease progresses, refractory motor, autonomic, and mental abnormalities may develop, which implies that there is progressive degeneration of striatal receptor mechanisms.

The clinical diagnosis of Parkinson's Disease is based on the presence of characteristic physical signs. The disease is known to be gradual in onset, slowly progressive, and variable in clinical manifestation. Evidence suggests that the striatal dopamine content declines to 20% below levels found in age-matched controls before symptoms occur.

Treatment of Parkinson's disease has been attempted with, inter alia, L-dopa (levodopa), which still is the gold standard for the therapy of Parkinson's Disease. Levodopa passes the blood-brain barrier as a precursor for dopamine and is then converted into dopamine in the brain. L-dopa improves the symptoms of Parkinson's Disease but may cause severe side effects. Moreover, the drug tends to lose its effectiveness after the first two to three years of treatment. After five to six years, only 25% to 50% of patients maintain improvement.

Furthermore a major drawback of currently utilized therapies for Parkinson's Disease is the eventual manifestation of the "fluctuation syndrome", resulting in "all-or-none" conditions characterized by alternating "on" periods of mobility with dyskinesias and "off" periods with hypokinesia or akinesia. Patients who display unpredictable or erratic "on-off" phenomena with oral anti-Parkinson therapy have a predictable beneficial response to i.v. administration of L-dopa and other dopamine agonists, suggesting that fluctuations in plasma concentrations of drug are responsible for the "on-off" phenomena. The frequency of "on-off" fluctuations has also been improved by continuous infusions of the dopamine receptor agonists apomorphine and lisuride. However, this mode of administration is inconvenient. Therefore, other modes of administration providing a more constant plasma level, such as topical administration, are beneficial and have been suggested in the past.

As mentioned above, one treatment approach for Parkinson's disease involves dopamine receptor agonists. Dopamine receptor agonists (sometimes also referred to as dopamine agonists) are substances which, while structurally different from dopamine, bind to different subtypes of dopamine receptors and trigger an effect which is comparable to that of dopamine. Due to the reduced side-effects, it is advantageous when the substances selectively bind to a subgroup of dopamine receptors, i.e. the D2 receptors.

One dopamine receptor agonist which has been used to treat the symptoms of Parkinson's Disease is rotigotine. It has mostly been used in the form of its hydrochloride. Rotigotine is the International Non-Proprietary Name (INN) of the compound (-)-5,6,7,8-tetrahydro-6-[propyl-[2-(2-thienyl)ethyl]-amino]1-naphthalenol having the structure shown below

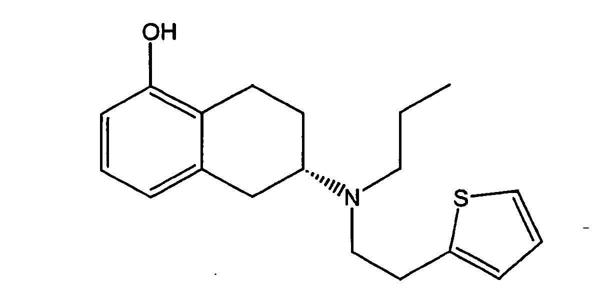

To date, various transdermal therapeutic systems (TTS) for the administration of rotigotine have been described. WO 94/07468 discloses a transdermal therapeutic system containing rotigotine hydrochloride as active substance in a two-phase matrix which is essentially formed by a hydrophobic polymer material as the continuous phase and a disperse hydrophilic phase contained therein and mainly containing the drug and hydrated silica. The silica enhances the maximum possible loading of the TTS with the hydrophilic salt. Moreover, the formulation of WO 94/07468 usually contains additional hydrophobic solvents, permeation-promoting substances, dispersing agents and, in particular, an emulsifier which is required to emulsify the aqueous solution of the active principle in the lipophilic polymer phase. A TTS, prepared by using such a system, has been tested in healthy subjects and Parkinson patients. The average drug plasma levels obtained by using this system were around 0.15 ng/ml with a 20 cm² patch containing 10 mg rotigotine. This level must be considered as too low to achieve a truly efficacious treatment or alleviation of symptoms related to Parkinson's disease.

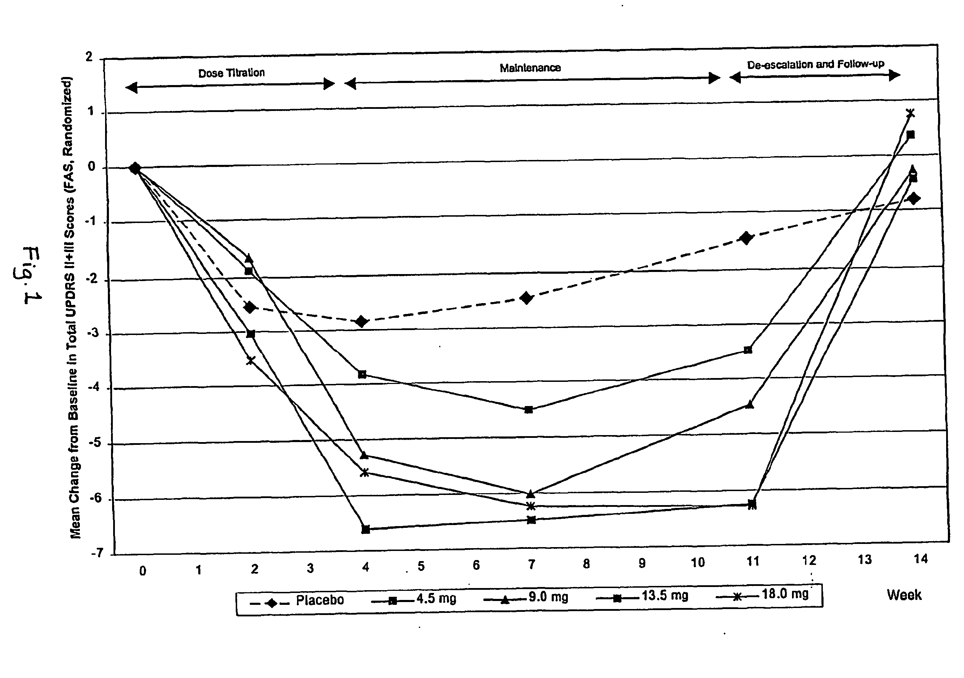

Various further transdermal therapeutic systems have been described in WO 99/49852. The TTS used in this patent application comprise a backing layer, inert with respect to the constituents of the matrix, a self-adhesive matrix layer containing an effective quantity of rotigotine or rotigotine hydrochloride and a protective film which is to be removed before use. The matrix system is composed of a non-aqueous polymer adhesive system, based on acrylate or silicone, with a solubility of rotigotine of at least 5% w/w. Said matrix is essentially free of inorganic silicate particles. In Examples 1 and 2 and in Figure 1 of WO 99/49852 two transdermal therapeutic systems are compared. These are based on acrylate or silicone adhesives, respectively. Figure 1 of WO 99/49852 shows that a silicone patch releases about the same amount of active principle through skin as an acrylate patch. This has been demonstrated by the almost identical drug flux rates in an in vitro model, independent of the adhesive test system employed. Therefore an identical flux rate through human skin was expected.

It should be noted that the drug content of the silicone patch used in WO 99/49852 was lower than the drug content of the acrylate patch. However, this merely reflects the difference in solubility of the drug in the respective polymeric silicone and acrylate adhesives used in Examples 1 and 2, respectively. The TTS used in both examples contained the drug at about its saturation solubility in the respective adhesive systems. While the acrylate system is able to dissolve more drug than the silicone system, silicone in turn allows for a better release of the drug to skin. As these two effects compensate each other, it has been thought that the acrylate and the silicone systems as used in WO 99/49852 are about equivalent in the obtainable drug plasma levels and, hence, in therapeutic efficacy.

Considering the rather discouraging experiences made with the silicone formulation of WO 94/07568, the acrylate-based TTS of Example 1 of WO 99/49852 has been subjected to clinical tests (safety and pharmacokinetic studies). The mean steady flux rate across human skin in vitro of this TTS amounted to 15.3 µg/cm²/h. However, it turned out that the plasma levels obtained using this TTS were still unsatisfactory and too low to allow for a really efficacious treatment of Parkinson's Disease. A 30 mg (20 cm²) patch only yielded a mean maximum plasma concentration of 0.12 ng/ml, while a 5 cm² patch containing 7.5 mg yielded a mean maximum plasma concentration of 0.068 ng/ml. Again, such values have to be considered as too low to provide a real therapeutic progress in the treatment of Parkinson's Disease. Thus, in summary, both the 20 cm² silicone patch of WO 94/07468 and the 20 cm² acrylate patch of WO 99/49852 failed to evoke sufficient drug plasma levels to provide a satisfactory therapeutic effectiveness.

In view of these experiences, it has been very surprising that a transdermal therapeutic system containing rotigotine in free base form in a silicone matrix could not only provide unexpectedly high plasma levels of rotigotine but also a significant therapeutic progress in the transdermal treatment of Parkinson's Disease. In particular, it has been unexpected that a transdermal therapeutic system having a size of as little as 10 or 20 cm² could provide for an effective treatment of Parkinson's Disease in a placebo-controlled clinical study, as indicated by an improvement in the Unified Parkinson's Disease Rating Scale (UPDRS) of 2 or more compared to a placebo treatment. In the context of this application, "placebo treatment" refers to a treatment with a transdermal therapeutic system of identical qualitative composition and according to the same therapy regimen but where the active ingredient (rotigotine) in the transdermal therapeutic system has been omitted.

It should be understood that the term "treatment" in the context of this application is meant to designate a treatment or alleviation of the symptoms of Parkinson's Disease, rather than a real causative treatment of Parkinson's Disease leading to a complete cure.

### Summary of the Invention

The present invention provides a silicone-based transdermal therapeutic system comprising a mixture of at least one high tack and at least one medium tack silicon pressure sensitive adhesive having an area of 10 to 40 cm² and containing 0.1 to 3.15 mg / cm² of rotigotine in the free base form as active ingredient wherein the silicone-based transdermal therapeutic system is suitable to effect an improvement, compared to a placebo treatment, of the condition of human Parkinson patients, measured according to the Unified Parkinson's Disease Rating Scale (UPDRS) parts II and III, of 2 units or more following administration for a time period of at least 7 weeks, preferably at least 11 weeks.

The silicone-based transdermal therapeutic system of the present invention contains a mixture of at least one high tack and at least one medium tack silicon pressure sensitive adhesive as the main component. Usually, the silicone compound will form a matrix in which the other components of the TTS are embedded. Moreover, the adhesive(s) should preferably be pharmaceutically acceptable in a sense that it is biocompatible, non-sensitizing and non-irritating to skin. Particularly advantageous silicone adhesives for use in the present invention should further meet the following requirements:
- Retained adhesive and cohesive properties in the presence of moisture or perspiration, under normal temperature variations,
- good compatibility with rotigotine as well as with the further excipients used in the formulation; in particular, the adhesive should not react with the amino group contained in rotigotine.

It has been shown that pressure sensitive adhesives of the type forming a soluble polycondensed polydimethylsiloxane (PDMS) / resin network, wherein the hydroxy endgroups are capped with e.g. trimethylsilyl (TMS) groups, are particularly useful in the practice of the present invention. Preferred adhesives of this kind are the BIO-PSA silicone pressure sensitive adhesives manufactured by Dow Corning, particularly the Q7-4201 and Q7-4301 qualities. However, other silicone adhesives may likewise be used.

Such a mixture of silicone adhesives comprising at least one high tack and at least one medium tack adhesive provides for the optimum balance between good adhesion and little cold flux. Excessive cold flux may result in a too soft patch which easily adheres to the package or to patient garments. Moreover, such a mixture of adhesives is particularly useful for obtaining an efficacious transdermal therapeutic system. A mixture of the aforementioned Q7-4201 (medium tack) and Q7-4301 (high tack) amine resistant silicone pressure sensitive adhesives in about equal amounts proved to be particularly useful in the practice of this invention.

In a further preferred embodiment, the silicone-based transdermal therapeutic system further includes a solubilizer. Several surfactant-like or amphiphilic substances may be used as solubilizers. They should be pharmaceutically acceptable and approved for use in medicaments. It is advantageous if the solubilizer also acts to improve the cohesion of the transdermal therapeutic system. A particularly preferred example of such a solubilizer is soluble polyvinylpyrrolidone.

Polyvinylpyrrolidone is commercially available, e.g. under the trademark Kollidon^{®} (Bayer AG). Other examples include copolymers of polyvinylpyrrolidone and vinyl acetate, polyethyleneglycol, polypropylene glycol, glycerol and fatty acid esters of glycerol or copolymers of ethylene and vinylacetate.

The silicone-based transdermal therapeutic system for use according to the present invention preferably contains less than 1 wt% of inorganic silicates, most preferably it is completely free from inorganic silicates.

The water content in the transdermal therapeutic systems for use in the present invention is preferably low enough so that no evaporation of water during preparation of the TTS is necessary. Typically, the water content in a freshly prepared patch is below 2%, more preferably 1 wt% or lower.

In a particularly preferred embodiment of the present invention, the transdermal therapeutic system has a surface area of 10 to 30 cm², more preferably 20 to 30 cm². It goes without saying that a TTS having a surface area of, say, 20 cm² is pharmacologically equivalent to and may be exchanged by two 10 cm² patches or four 5 cm² patches having the same drug content per cm². Thus, the surface areas as indicated in this application should be understood to refer to the total surface of all TTSs simultaneously administered to a patient.

Providing and applying one or several transdermal therapeutic systems according to the invention has the pharmacological advantage over oral therapy that the attending physician can titrate the optimum dose for the individual patient relatively quickly and accurately, e.g. by simply increasing the number or size of patches given to the patient. Thus, the optimum individual dosage can often be determined after a time period of only about 3 weeks with low side effects.

A preferred content of rotigotine per patch is in the range of 0.1 to 2.0 mg / cm². Still more preferred are 0.4 to 1.5 mg rotigotine per cm². If a 7 day patch is desired, higher drug contents will generally be required. A rotigotine content in the range of about 0.4 to 0.5 wt% has been found to be particularly advantageous in that it provides the optimum usage of the drug contained in the TTS, i.e. there is only very little residual drug content in the TTS after administration. The apparent dose administered by using such a TTS usually is 50% or more and may be as high as 80-90% of the drug amount originally contained in the TTS.

The fact that the silicone-based transdermal therapeutic system according to this invention is able to provide a significant therapeutic effect against symptoms of Parkinson's Disease even at surface areas of 10 to 30 cm² and particularly as little as 10 or 20 cm² and at low drug contents of about 0.4 to 0.5 mg/cm², particularly about 0.45 g/cm², must be considered as a further particular benefit provided by the present invention.

The transdermal therapeutic system of the present invention usually is a patch having a continuous adhesive matrix in at least its center portion containing the drug. However, transdermal equivalents to such patches are likewise comprised by the present invention, e.g. an embodiment where the drug is in an inert but non-adhesive silicone matrix in the center portion of the TTS and is surrounded by an adhesive portion along the patch edges.

In a further aspect, this invention relates to the use of a silicone-based transdermal therapeutic system having an area of 10 to 40 cm² and containing 0.1 to 3.15 mg / cm² of rotigotine as active ingredient, for preparing as anti-Parkinson medicament which improves the condition of the patient, measured according to the Unified Parkinson's Disease Rating Scale (UPDRS) parts II and III, vis á vis placebo treatment, by about 2 units or more over a time period of at least 7 weeks administration. Maintenance of this UPDRS score improvement for up to 7 weeks has been shown as well. Thus, improving the UPDRS (parts II+III) score over placebo by at least 2 units following administration for 7, preferably 11 weeks forms a particularly beneficial aspect of the present invention.

Unless expressly indicated otherwise, any references to rotigotine in the context of this invention and the claims of this application mean rotigotine in the form of its free base. In some cases traces of rotigotine hydrochloride may be contained in rotigotine but these traces do typically not exceed 5 wt%, based on the amount of the free base. More preferably the content of hydrochloride impurities should be less than 2 wt%, even more preferably less than 1%, and most preferably the rotigotine used in the present invention contains less than 0.1 wt% or no hydrochloride impurities at all.

Extensive clinical trials using the transdermal therapeutic system described herein have shown that it is surprisingly possible to achieve and warrant a constant stimulation of the dopamine receptors of Parkinson patients, resulting in a notable improvement in the clinically relevant UPDRS for a time period of at least 7 weeks. More specifically, clinical studies using a patch according to the preparation example as given herein have resulted in the following improvements in the FAS UPDRS (part II and III) score following administration for 11 weeks:

| Patch Size | Amount of rotigotine | Improvement in UPDRS over Placebo Treatment | p (one sided) |
|---|---|---|---|
| 10 cm² | 4.5 mg | - 2.148 | 0.0393 |
| 20 cm² | 9.0 mg | - 3.123 | 0.0063 |
| 30 cm² | 13.5 mg | - 4.909 | 0.0000 |
| 40 cm² | 18.0 mg | - 5.035 | 0.0000 |

The abbreviation FAS stands for "Full Analysis Sent" and thus designates an analysis including all patients who were included in the study. The UPDRS score and the study design is explained in more detail in the Clinical Trials Example below. Fig. 1 is a graph illustrating the mean change from base line in UPDRS (II + III) total scores from day 0 to end of treatment in this study. This figure compares the effects of a treatment according to the present invention with a placebo treatment. Statistically significant improvements can particularly be observed for patches having an area of 20 cm² or more, though even the effect of the 10 cm² patch must be considered as an improvement. The value indicated as p in the above table represents the one-sided p-value obtained from a statistical evaluation of the trial data.

While an improvement in the UPDRS scale of 2, compared to placebo, may already be called a success, an improvement of 3, 4 or even 5 or more units would even more represent a therapeutic progress and, as such, form a preferred aspect of the present invention.

Additional tests including pharmacokinetics, dose-activity relationship, compliance, and drug safety confirmed the therapeutic usefulness of the silicone-based transdermal therapeutic system used herein.

The invention and the best mode for carrying it out will be explained in more detail in the following non-limiting

### examples.

### Preparation Example

A transdermal therapeutic system using a combination of silicone-type pressure sensitive adhesives was prepared as follows.

(-)-5,6,7,8-tetrahydro-6-[propyl-[2-(2-thienyl)ethyl]-amino]-1-naphthalenol hydrochloride (rotigotine hydrochloride, 150 g) was added to a solution of 17.05 g NaOH in 218 g ethanol (96%). The resulting mixture was stirred for approximately 10 minutes. Then 23.7 g of sodium phosphate buffer solution (8.35 g Na₂HPOP₄x2H₂O and 16.07 g NaH₂PO₄x2H₂O in 90.3 g water) was added. Insoluble or precipitated solids were separated from the mixture by filtration. The filter was rinsed with 60.4 g ethanol (96%) to obtain a particle-free ethanolic solution of rotigotine in the form of the free base.

The rotigotine free base solution (346.4 g) in ethanol (35% w/w) was mixed with 36.2 g ethanol (96%). The resulting solution was mixed with 109 g of an ethanolic solution containing 25 wt% polyvinylpyrrolidone (KOLLIDON^{®} 90F), 0.077 wt% aqueous sodium bisulfite solution (10 wt%), 0.25 wt% ascorbyl palmitate, and 0.63 wt% DL-alpha-tocopherol until homogenous. To the mixture, 817.2 g of an amine resistant high tack silicone adhesive (BIO-PSA^{®} Q7-4301 mfd. by Dow Corning) (74 wt% solution in heptane), 851.8 g of an amine resistant medium tack silicone adhesive (BIO-PSA^{®} Q7-4201 mfd. by Dow Corning) (71 wt% solution in heptane), and 205.8 g petrol ether (heptane) were added, and all components were stirred until a homogenous dispersion was obtained.

The dispersion was coated onto a suitable polyester release liner (SCOTCHPAK^{®} 1022) with a suitable doctor knife and the solvents were continuously removed in a drying oven at temperatures up to 80°C for about 30 min to obtain a drug-containing adhesive matrix of 50 g/m² coating weight. The dried matrix film was laminated with a polyester-type backing foil (SCOTCHPAK^{®} 1109). The individual patches were punched out of the complete laminate in the desired sizes (e.g. 10 cm², 20 cm², 30 cm²) and sealed into pouches under the flow of nitrogen.

The following table shows the composition in mg/20 cm² of a transdermal therapeutic system according to the present invention containing a combination of two silicone-type PSA.

| Composition Components | Amount (mg) |
|---|---|
| Rotigotine Base | 9.00 |
| Polyvinylpyrrolidone | 2.00 |
| Silicone BIO-PSA^{®} Q7-4301 | 44.47 |
| Silicone BIO-PSA^{®} Q7-4201 | 44.46 |
| Ascorbyl palmitate | 0.02 |
| DL-alpha Tocopherol | 0.05 |
| Sodium metabisulfite | 0.0006 |
| Matrix coating weight | 50 g/m² |

### Clinical Trials

### Methods:

The rotigotine TTS as prepared in the above preparation example has been tested in multicenter, placebo-controlled, double-blind, randomised clinical trials involving more than 300 Parkinson patients, and proven to yield an effective alleviation of the symptoms of Parkinson's Disease in patients suffering from this disease upon prolonged administration times (11 weeks, of which 4 weeks were a titration period and 7 weeks were the maintenance period), once daily administration). The patients were not receiving other dopaminergic medications at that time.

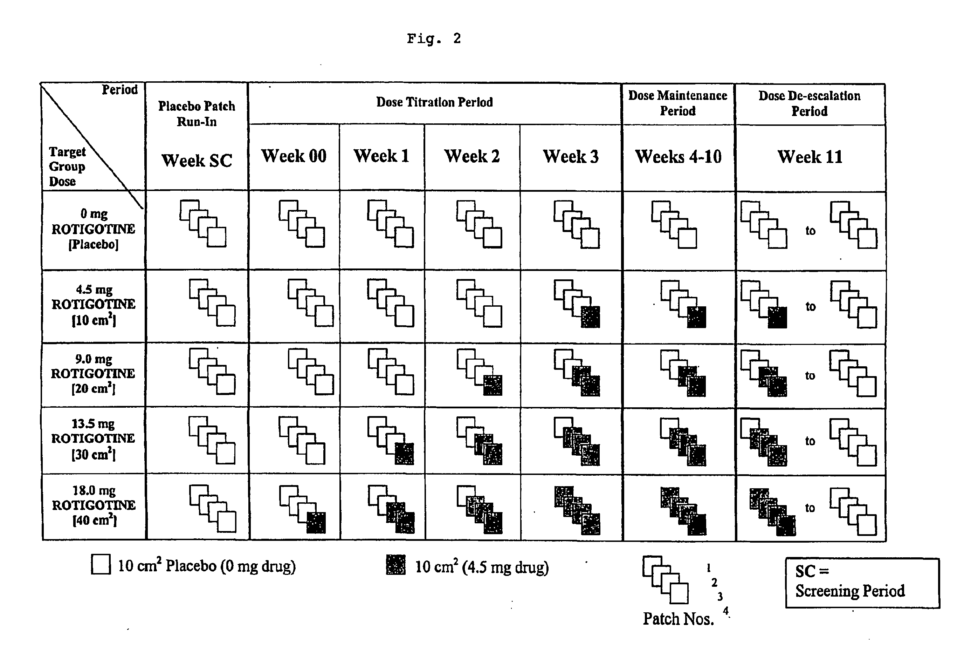

After a 4-7 day open-label placebo run-in period, 329 patients were randomized to placebo or one of four rotigotine daily dosages (drug content in patch 4.5 mg, 9.0 mg, 13.5 mg or 18 mg), and followed for a 4-week dose-titration period, a 7-week dose-maintenance period, a 1-week dose de-escalation period and a 2-week safety follow-up period in a double-blind fashion. The study design (therapy plan) is illustrated in more detail in Fig. 2.

The prespecified primary efficacy outcome was a change in the Activities of Daily Living and Motor components of the generally accepted Unified Parkinson's Disease Rating Scale (UPDRS II/III) between baseline and the last evaluation on treatment (week 11).

Parts II and III of the UPDRS measure and rate the following clinical parameters in Parkinson patients:

### II. ACTIVITES OF DAILY LIVING

A. Speech

| | |
|---|---|
| 0= | Normal. |
| 1= | Mildly affected. No difficulty being understood. |
| 2= | Moderately affected. Sometimes asked to repeat statements. |
| 3= | Severely affected. Frequently asked to repeat statements. |
| 4= | Unintelligible most of the time. |

B. Salivation

| | |
|---|---|
| 0= | Normal. |
| 1= | Slight but definite excess of saliva in mouth; may have nighttime drooling. |
| 2= | Moderately excessive saliva; may have minimal drooling. |
| 3= | Marked excess of saliva with some drooling. |
| 4= | Marked drooling, requires constant tissue or handkerchief. |

C. Swallowing

| | |
|---|---|
| 0= | Normal. |
| 1= | Rare choking. |
| 2= | Occasional choking. |
| 3= | Requires soft food. |
| 4= | Requires NG tube or gastronomy feeding. |

D. Handwriting

| | |
|---|---|
| 0= | Normal. |
| 1= | Slightly slow or small. |
| 2= | Moderately slow or small; all words are legible. |
| 3= | Severely affected; not all words are legible. |
| 4= | The majority of words are not legible. |

E. Cutting food and handling utensils

| | |
|---|---|
| 0= | Normal. |
| 1= | Somewhat slow and clumsy, but no help needed. |
| 2= | Can cut most foods, although clumsy and slow, some help needed. |
| 3= | Food must be cut by someone, but can still feed slowly. |
| 4= | Needs to be fed. |

F. Dressing

| | |
|---|---|
| 0= | Normal. |
| 1= | Somewhat slow, but no help needed. |
| 2= | Occasional assistance with buttoning, getting arms in sleeves. |
| 3= | Considerable help required, but can do some things alone. |
| 4= | Helpless. |

G. Hygiene

| | |
|---|---|
| 0= | Normal. |
| 1= | Somewhat slow, but no help needed. |
| 2= | Needs help to shower or bathe; or very slow in hygienic care. |
| 3= | Requires assistance for washing, brushing teeth, combing hair, going to bathroom. |
| 4= | Foley catheter or other mechanical aids. |

H. Turning in bed and adjusting bed clothes

| | |
|---|---|
| 0= | Normal. |
| 1= | Somewhat slow and clumsy, but no help needed. |
| 2= | Can turn alone or adjust sheets, but with great difficulty. |
| 3= | Can initiate, but not turn or adjust sheets alone. |
| 4= | Helpless. |

I. Falling (unrelated to freezing)

| | |
|---|---|
| 0= | None. |
| 1= | Rare falling. |
| 2= | Occasionally falls, less than once per day. |
| 3= | Falls an average of once daily. |
| 4= | Falls more than once daily. |

J. Freezing when walking

| | |
|---|---|
| 0= | None. |
| 1= | Rare freezing when walking; may have start hesitation. |
| 2= | Occasional freezing when walking. |
| 3= | Frequent freezing. Occasionally falls from freezing. |
| 4= | Frequent falls from freezing. |

K. Walking

| | |
|---|---|
| 0= | Normal. |
| 1= | Mild difficulty. May not swing arms or may tend to drag leg. |
| 2= | Moderate difficulty, but requires little or no assistance. |
| 3= | Severe disturbance of walking, requiring assistance. |
| 4= | Cannot walk at all, even with assistance. |

L. Tremor (Symptomatic complaint of tremor in any part of body.)

| | |
|---|---|
| 0= | Absent. |
| 1= | Slight and infrequently present. |
| 2= | Moderate; bothersome to patient. |
| 3= | Severe; interferes with many activities. |
| 4= | Marked; interferes with most activities. |

M. Sensory complaints related to parkinsonism

| | |
|---|---|
| 0= | None. |
| 1= | Occasionally has numbness, tingling, or mild aching. |
| 2= | Frequently has numbness, tingling, or aching; not distressing. |
| 3= | Frequent painful sensations. |
| 4= | Excruciating pain. |

### III. MOTOR EXAMINATION

N. Speech

| | |
|---|---|
| 0= | Normal. |
| 1= | Slight loss of expression, diction and/or volume. |
| 2= | Monotone, slurred but understandable; moderately impaired. |
| 3= | Marked impairment, difficult to understand. |
| 4= | Unintelligible. |

O. Facial Expression

| | |
|---|---|
| 0= | Normal. |
| 1= | Minimal hypomimia, could be normal "Poker Face". |
| 2= | Slight but definitely abnormal diminution of facial expression. |
| 3= | Moderate hypomimia; lips parted some of the time. |
| 4= | Masked or fixed facies with severe or complete loss of facial expression; lips parted ¼ inch or more. |

P. Tremor at rest (head, upper and lower extremities)

| | |
|---|---|
| 0= | Absent. |
| 1= | Slight and infrequently present. |
| 2= | Mild in amplitude and persistent. Or moderate in amplitude, but only intermittently present. |
| 3= | Moderate in amplitude and present most of the time. |
| 4= | Marked in amplitude and present most of the time. |

Q. Action or Postural Tremor of hands

| | |
|---|---|
| 0= | Absent. |
| 1= | Slight; present with action. |
| 2= | Moderate in amplitude; present with action. |
| 3= | Moderate in amplitude with posture holding as well as action. |
| 4= | Marked in amplitude; interferes with feeding. |

R. Rigidity (Judged on passive movement of major joints with patient relaxed in sitting position. Cogwheeling to be ignored).

| | |
|---|---|
| 0= | Absent. |
| 1= | Slight or detectable only when activated by mirror or other movements. |
| 2= | Mild to moderate. |
| 3= | Marked, but full range of motion easily achieved. |
| 4= | Severe, range of motion achieved with difficulty. |

S. Finger Taps (Patient taps thumb with index finger in rapid succession.)

| | |
|---|---|
| 0= | Normal. |
| 1= | Mild slowing and/or reduction in amplitude. |
| 2= | Moderately impaired. Definite and early fatiguing. May have occasional arrests in movement. |
| 3= | Severely impaired. Frequent hesitation in initiating movements or arrests in ongoing movement. |
| 4= | Can barely perform the task. |

T. Hand Movements (Patient opens and closes hands in rapid succession.)

| | |
|---|---|
| 0= | Normal. |
| 1= | Mild slowing and/or reduction in amplitude. |
| 2= | Moderately impaired. Definite and early fatiguing. May have occasional arrests in movement. |
| 3= | Severely impaired. Frequent hesitation in initiating movements or arrests in ongoing movement. |
| 4= | Can barely perform the task. |

U. Rapid Alternating Movements of Hands (Pronation-supination movements of hands, vertically and horizontally, with as large an amplitude as possible, both hands simultaneously.)

| | |
|---|---|
| 0= | Normal. |
| 1= | Mild slowing and/or reduction in amplitude. |
| 2= | Moderately impaired. Definite and early fatiguing. May have occasional arrests in movement. |
| 3= | Severely impaired. Frequent hesitation in initiating movements or arrests in ongoing movement. |
| 4= | Can barely perform the task. |

V. Leg Ability (Patient taps heel on the ground in rapid succession picking up entire leg. Amplitude should be at least 3 inches.)

| | |
|---|---|
| 0= | Normal. |
| 1= | Mild slowing and/or reduction in amplitude. |
| 2= | Moderately impaired. Definite and early fatiguing. May have occasional arrests in movement. |
| 3= | Severely impaired. Frequent hesitation in initiating movements or arrests in ongoing movement. |
| 4= | Can barely perform the task. |

W. Arising from Chair (Patient attempts to rise from a straightbacked chair, with arms folded across chest.)

| | |
|---|---|
| 0= | Normal. |
| 1= | Slow; or may need more than one attempt. |
| 2= | Pushes self up from arms of seat. |
| 3= | Tends to fall back and may have to try more than one time, but can get up without help. |
| 4= | Unable to arise without help. |

X. Posture

| | |
|---|---|
| 0= | Normal erect. |
| 1= | Not quite erect, slightly stooped posture; could be normal for older person. |
| 2= | Moderately stooped posture, definitely abnormal; can be slightly leaning to one side. |
| 3= | Severely stooped posture with kyphosis; can be moderately leaning to one side. |
| 4= | Marked flexion with extreme abnormality of posture. |

Y. Gait

| | |
|---|---|
| 0= | Normal. |
| 1= | Walks slowly, may shuffle with short steps, but no festination (hastening steps) or propulsion. |
| 2= | Walks with difficulty, but requires little or no assistance; may have some festination, short steps, or propulsion. |
| 3= | Severe disturbance of gait, requiring assistance. |
| 4= | Cannot walk at all, even with assistance. |

Z. Postural Stability (Response to sudden, strong posterior displacement produced by pull on shoulders while patient erect with eyes open and feet slightly apart. Patient is prepared.)

| | |
|---|---|
| 0= | Normal. |
| 1= | Retropulsion, but recovers unaided. |
| 2= | Absence of postural response; would fall if not caught by examiner. |
| 3= | Very unstable, tends to lose balance spontaneously. |
| 4= | Unable to stand without assistance. |

AA. Body Bradykinesia and Hypokinesia (Combining slowness, hesitancy, decreased armswing, small amplitude, and poverty of movement in general.)

| | |
|---|---|
| 0= | None. |
| 1= | Minimal slowness, giving movement a deliberate character; could be normal for some persons. Possibly reduced amplitude. |
| 2= | Mild degree of slowness and poverty of movement which is definitely abnormal. Alternatively, some reduced amplitude. |
| 3= | Moderate slowness, poverty or small amplitude of movement. |
| 4= | Marked slowness, poverty or small amplitude of movement. |

The total UPDRS score is determined from the individual scores as follows:

First, a baseline value is determined for each patient participating in the study. This is done by summing up the individual scores of the UPDRS part II and III parameters at day 0, i.e. before treatment. Any determinations of the UPDRS score in the course of treatment will then be compared to this baseline value and changes relative to baseline value are recorded. Finally the mean improvement in UPDRS II+III at Day 77 (week 11) relative to baseline will be determined by averaging over all trial subjects. The resulting value is designated as the FAS (Full Analysis Set) Randomized Mean Change from Baseline Value in Total UPDRS Score (II + III) and is plotted as the y-axis in Figure 1. The term "randomized" refers to the fact that the patients were randomized to the different pre-defined doses in advance.

Patients suffering from Parkinson's Disease are known to experience a relatively strong placebo effect, i.e. even a placebo treatment improves the UPDRS score of Parkinson patients to some extent. It is therefore important to compare any effects of drug treatment with the level of UPDRS improvement attained by a placebo treatment of the same length. The final evaluation of improvement is therefore made relative to the effect of a placebo treatment of the same duration.

### Results:

There was a significant, dose-related improvement in UPDRS II/III scores between baseline and week 11 when applying the TTS according to the present invention, particularly for the 9.0, 13.5, and 18 mg groups, compared to placebo. This result is apparent from Figure 1 and the following table:

| Patch Size | Amount of rotigotine | Improvement in Mean Total UPDRS II+III over Placebo Treatment at week 11 | p (one sided) |
|---|---|---|---|
| 10 cm² | 4.5 mg | - 2.148 | 0.0393 |
| 20 cm² | 9.0 mg | - 3.123 | 0.0063 |
| 30 cm² | 13.5 mg | - 4.909 | 0.0000 |
| 40 cm² | 18.0 mg | - 5.035 | 0.0000 |

Rotigotine, when administered using the inventive TTS, was generally well tolerated. Application site skin reactions were generally mild and occurred with placebo patches, but were more common among subjects randomized to the higher dosage groups. There were no differences among the groups regarding vital signs, laboratory tests and ECGs.

### Conclusions:

The above results show for the first time in a placebo-controlled study that a dopamine agonist (rotigotine), administered transdermally and once daily by a specific TTS, produces clinical improvement with satisfactory tolerability and safety in patients with early Parkinson's Disease.

Such a result could neither be obtained with the acrylic transdermal therapeutic system of WO 99/49852 nor with the silicone transdermal therapeutic system of WO 94/07468. Before this background, this result must be viewed as particularly surprising and beneficial for Parkinson patients.

## Claims

1. A silicone-based transdermal therapeutic system comprising a mixture of at least one high tack and at least one medium tack silicone pressure sensitive adhesive as the main adhesive components having an area of 10 to 40 cm² and containing 0.1 to 3.15 mg / cm² of rotigotine in the free base form as active ingredient, wherein the silicone-based transdermal therapeutic system is suitable to effect an improvement, vis a vis placebo treatment, in the condition of a human Parkinson patient, measured according to the Unified Parkinson's Disease Rating Scale (UPDRS) parts II and III, of 2 units or more following administration for at least 7 weeks.

2. The silicone-based transdermal therapeutic system according to claim 1 wherein the silicone-based transdermal therapeutic system further includes a solubilizer.

3. The silicone-based transdermal therapeutic system according to claim 2 wherein the solubilizer is polyvinylpyrrolidone.

4. The silicone-based transdermal therapeutic system according to any of the preceding claims wherein the silicone-based transdermal therapeutic system contains less than 1 wt% of inorganic silicates.

5. The silicone-based transdermal therapeutic system according to claim 4 wherein the silicone-based transdermal therapeutic system is free from inorganic silicates.

6. The silicone-based transdermal therapeutic system according to any of the preceding claims wherein the transdermal therapeutic system has an area of 10 to 30 cm².

7. The silicone-based transdermal therapeutic system according to any of the preceding claims wherein the transdermal therapeutic system contains 0.1 to 1.5 mg / cm² of rotigotine.

8. The silicone-based transdermal therapeutic system according to claim 1 wherein the transdermal therapeutic system is a patch having an area of 10 to 30 cm² and a content of rotigotine of 0.4 to 0.5 mg/cm² in an adhesive silicone-based matrix.

## Patentansprüche

1. Auf Silikon basierendes, transdermales, therapeutisches System, umfassend eine Mischung von zumindestens einem hoch-klebrigen und mindestens einem mittel-klebrigen druckempfindlichen Silikon-Klebemittel als Haupt-Klebemittelkomponenten, mit einer Oberfläche von 10 bis 40 cm² und enthaltend 0,1 bis 3,15 mg/cm² Rotigotin in freier Basenform als Wirkstoff, worin das auf Silikonbasierende, transdermale, therapeutische System geeignet ist, um eine Verbesserung des Zustands eines menschlichen Parkinson-Patienten im Vergleich zu einer Placebo-Behandlung von 2 Einheiten oder mehr nach der Verabreichung für mindestens 7 Wochen herbeizuführen, gemessen gemäß dem Unified Parkinson Disease Rating Scale (UPDRS) Teile II und III,.

2. Auf Silikon basierendes, transdermales, therapeutisches System gemäß Anspruch 1, worin das auf Silikon basierende, transdermale, therapeutische System ferner einen Löslichkeitsvermittler umfasst.

3. Auf Silikon basierendes, transdermales, therapeutisches System gemäß Anspruch 2, worin der Löslichkeitsvermittler Polyvinylpyrrolidon ist.

4. Auf Silikon basierendes, transdermales, therapeutisches System gemäß irgendeinem der vorhergehenden Ansprüche, worin das auf Silikon basierende, transdermale, therapeutische System weniger als 1 Gew.% anorganische Silikate umfasst.

5. Auf Silikon basierendes, transdermales, therapeutisches System gemäß Anspruch 4, worin das auf Silikon basierende, transdermale, therapeutische System keine anorganischen Silikate enthält.

6. Auf Silikon basierendes, transdermales, therapeutisches System gemäß irgendeinem der vorhergehenden Ansprüche, worin das transdermales, therapeutisches System eine Fläche von 10 bis 30 cm² aufweist.

7. Auf Silikon basierendes, transdermales, therapeutisches System gemäß irgendeinem der vorhergehenden Ansprüche, worin das transdermales, therapeutisches System 0,1 bis 1,5 mg/cm² Rotigotin enthält.

8. Auf Silikon basierendes, transdermales, therapeutisches System gemäß Anspruch 1, worin das transdermales, therapeutisches System ein Pflaster mit einer Fläche von 10 bis 30 cm² und einem Rotigotingehalt von 0,4 bis 0,5 mg/cm² in einer auf Silikon basierenden Klebemittelmatrix ist.

## Revendications

1. Système thérapeutique transdermique à base de silicone, comprenant un mélange d'au moins un adhésif au silicone sensible à la pression, à forte adhésivité, et d'au moins un adhésif au silicone sensible à la pression, à adhésivité moyenne, comme composants adhésifs principaux, ayant une surface de 10 à 40 cm² et contenant comme principe actif de 0,1 à 3,15 mg/cm² de rotigotine sous forme de base libre, dans lequel le système thérapeutique transdermique à base de silicone est apte à entraîner une amélioration, par rapport à un traitement par placebo, de la condition d'un patient humain atteint de maladie de Parkinson, mesurée selon l'échelle d'évaluation unifiée pour la maladie de Parkinson (UPDRS) parties II et III, de 2 unités ou plus à la suite d'une administration sur au moins 7 semaines.

2. Système thérapeutique transdermique à base de silicone selon la revendication 1, dans lequel le système thérapeutique transdermique à base de silicone comprend en plus un solubilisant.

3. Système thérapeutique transdermique à base de silicone selon la revendication 2, dans lequel le solubilisant est la polyvinylpyrrolidone.

4. Système thérapeutique transdermique à base de silicone selon l'une quelconque des revendications précédentes, dans lequel le système thérapeutique transdermique à base de silicone contient moins de 1 % pondéral de silicates inorganiques.

5. Système thérapeutique transdermique à base de silicone selon la revendication 4, dans lequel le système thérapeutique transdermique à base de silicone est exempt de silicates inorganiques.

6. Système thérapeutique transdermique à base de silicone selon l'une quelconque des revendications précédentes, dans lequel le système thérapeutique transdermique a une surface de 10 à 30 cm².

7. Système thérapeutique transdermique à base de silicone selon l'une quelconque des revendications précédentes, dans lequel le système thérapeutique transdermique contient de 0,1 à 1,5 mg/cm² de rotigotine.

8. Système thérapeutique transdermique à base de silicone selon la revendication 1, dans lequel le système thérapeutique transdermique est un "patch" (dispositif transdermique) ayant une surface de 10 à 30 cm² et un dosage en rotigotine de 0,4 à 0,5 mg/cm² dans une matrice adhésive à base de silicone.
